# EUROPEAN PATENT APPLICATION

(11) **EP 1 358 856 A1**
(43) Date of publication of application: **05.11.2003**
(21) Application number: 03425262.7
(22) Date of filing: 29.04.2003
(51) Int. Cl.: A61D 19/02, A61M 5/315

(54) **Device for intrauterine, intrabutal and intraperitoneal transfer of biological material**

(30) Priority: 29.04.2002 IT RM20020230
(71) Applicant: UNIVERSITA DEGLI STUDI DI SIENA, 53100 Siena (IT)
(72) Inventor: Inaudi, Pieraldo, Universita' degli Studi di Siena, 53100 Siena (IT)
(74) Representative: Iannone, Carlo Luigi

(57) **Abstract**

The invention relates to a device (1; 100) for intrauterine and/or intratubal and/or intraperitoneal transfer of biological material, comprising a syringe element (2; 102), provided with a piston (4; 104) and means for drawing and introducing biological material, rotative means (8, 108) for shifting said piston (4; 104) of the syringe element (2; 102), and restraint means for shifting the piston (4; 104), thus allowing a linear and non rotative shifting of the same piston.

## Description

### Field of the Invention

The present invention relates to a device for intrauterine and/or intratubal and/or intraperitoneal transfer of biological material.

Particularly the invention relates to a syringe device for intrauterine and/or intratubal and/or intraperitoneal transfer of biological material, seminal fluid or embryos, which allows a steady and moderate rate emission of the same, thus reducing contamination risk.

### State of the Art

The device can be used in the field of both human and animal artificial insemination.

It appears from the census data of 1991 in Italy, that about 18.000 couples out of 60.000 marriages are subfertile or infertile. Each year about 8.000 new couples turn to a medical assistant to attain pregnancy. By now, the causes of infertility are split out at 50% among man and woman.

After accurate diagnosis it is frequent to turn to drugs that induce ovulation or superovulation, in association with the use of one among the assisted reproduction techniques.

Among these techniques, the widely used at outpatient level is intrauterine insemination of motile sperm cells. Other methods of *in vivo* insemination (intracorporeal fecundation) are intratubal and intraperitoneal insemination that are less frequently used than intrauterine insemination.

Nevertheless also these techniques utilize prepared motile sperm cells and are executed in outpatient room.

To allow sperm cells insertion into uterus, tube or peritoneum, cells must be prepared, isolating mobile and normal shaped form ones from the other spermatozoa and from other typical cells of seminal fluid (leucocytes, ephitelial cells, germinal cells, etc.), as well as from the same seminal fluid.

To this aim two methods have been prepared mainly, i.e. ascendent spontaneous migration method (Swim-up) to be used prevalently with normal spermigrams or patient afflicted with moderate oligoasthenospermatism and density gradient percolation method (ex Percoll) to be used with patients afflicted with medium-serious oligoasthenoteratospermatism.

In 1999 OMS has codified the two methods, reporting them in a new edition of WHO Laboratory Manual.

One of the rules which guided method's choice to be used in sperma cells preparation, was the one based on the capability of recovering the maximum number as possible of vital spermatozoa.

Recent data have pointed out that also a modest quantity of motile spermatozoa may be sufficient to ensure infertility treatments through assisted reproduction techniques to be successful. Then, "swim up" technique, which usually offers a lower recovery of spermatozoa, in terms of percentage and not of absolute number, than density gradient centrifugation method, can find its appliance also with patients afflicted with moderate oligoasthenospermatism.

The present basic citeria has shifted from a maximum recovery one to a suitable recovery to the use technique, using the less traumatic technique for spermatozoa.

It is necessary to consider that sperm can be carrier of infections and on the other hand, in spite of all the precautions, it can become infected before its insertion into uterus.

On the basis of the above, it would be desirable to have at disposal a reliable device which allows a suitable recovery of spermatozoa, without causing any trauma, such as those resulting from centrifugation and thus reducing at the same time contamination risks.

According to the present invention, it has been verified the efficacy of a device based on "swim-up" method simplified, in comparison with classic method and which allows spermatozoa recovery without any centrifugation.

As it is well-known, classic method consists in laying seminal fluid by sterilised pipette into a conical test tube and thus slowly stratifying a culture medium on the same seminal fluid.

The test tube is closed, inclined at 45° and then incubated at 37°C for 60 minutes. The supernatant, containing spermatozoa that migrated, is removed by pipette and transferred in a sterile conical test tube.

Afterwards, culture medium is added to the saving supernatant and the mixture is centrifuged. The fluid is drawn through slow aspiration by means of a pipette and then removed, meanwhile "pellet" is suspended in a culture medium and maintained at 37°C until the moment of the same use.

It is also well-known that the simplified method consists in using a syringe in place of test tubes and it does not provide centrifugation application.

By means of a syringe, first, a culture medium and after seminal fluid are aspirated in such a way to make two layers. The syringe is inclined at 45° and incubated at 37°C for 60 minutes. Then, the seminal fluid is ejected from the syringe and the remaining culture medium containing motile spermatozoa is maintained at 37°C until the use.

Therefore, through the simplified method, sperm contamination risk is reduced as work is done by means of a closed syringe and not by test-tubes. Besides, traumatic stress to spermatozoa are reduced to their lowest, since no centrifugation is applied.

Nevertheless, the use of a traditional syringe produces some problems, that are due to the mechanical stress spermatozoa are subjected to, when they pass through the needle. Furthermore, as the action exerted on the piston often causes a jerkily and not progressive feed, syringe's contents is ejected at high speed in relation to the type of material at issue with possible traumatic risk to spermatozoa or embryos.

In the light of the above, it appears to be evident the need to have at disposal a device, which solves the disadvantage above mentioned, allowing a steady and moderate rate emission of biological material, thus reducing sperm contamination risks.

According to the present invention, these and other results are achieved, through carrying out an innovative device for the intrauterine, intratubal or intraperitoneal transfer of biological material, which fulfils the requirements above mentioned, assisting both spermatozoa recovery phase and intrauterine insemination phase, as well as allowing intrauterine, intratubal or intraperitoneal transfer of pre-embryos and embryos under less traumatic conditions.

### Summary of the invention

The object of the present invention is a device for the intrauterine and/or intratubal and/or intraperitoneal transfer of biological material comprising a syringe element, provided with a piston and means for drawing and introducing biological material, rotative means for shifting said piston of the syringe element, and restraint means for shifting the piston, thus allowing a linear and non rotative shifting of the same piston.

In a first embodiment of the device according to the invention, said device comprises the syringe body, within which a chamber is created, along said chamber a piston sliding, in front of said piston a catheter or similar element is provided, and provided, at the opposite end, with a threaded ring nut, rotatably mounted on said piston and acting with a corresponding thread provided outside said piston, and restraint means for the piston sliding, preventing the rotation under the ring nut action, thus allowing only its longitudinal sliding along the chamber of said sirynge.

Particularly, according to the invention, at the rear of said chamber of the syringe body, a positioning element is provided, having step means, acting with elastic fingers realised in a end element, provided outside the ring nut with respect to said syringe body and passing within the same ring nut.

In a second embodiment of the device according to the invention, said device includes a body, within which an actuating element or piston slides, that can be coupled in front of the rear end of a standard syringe piston, and couple at the opposite end with a dosage rotating ring nut, by interaction between corresponding threads, means being provided preventing the rotation of the actuating element with respect to the body due to the ring nut action, thus allowing only its longitudinal advancement.

Preferably, according to the invention, coupling between the front end of the actuating element and the rear end of the syringe piston is obtained by an elastic clip.

Particularly, it can be further provided a coupling ring nut between the front end of the actuating element and the rear end of the syringe piston, that is inserted outside the syringe and block said elastic clip.

Still according to the invention, said rotating dosage ring nut is maintained in position by plate means.

Furthermore, according to the invention, said actuating element is provided with a longitudinal groove, interacting with pin means or like, sliding within the same, thus preventing the rotation of the actuating means due to the action of the ring nut, and allowing only its longitudinal sliding.

Always according to the invention, can be provided different kind of coupling ring nut and elastic clip in function of the kind of standard syringe.

The catheter can be provided with round shaped tip having side apertures to reduce shocks risk both to uterine structures such as canalis cervicis, ostium internum and endometrium, and to biological material ejected from the syringe, since the peculiar conformation of the tip with side leakages reduces the pressures under which biological material is submitted, in comparison with a catheter having frontal aperture.

Moreover, the catheter can include echoreflecting material which allows to display the catheter through ultrasounds and then to carry out uterine transfer under echography guide. In fact, it is common knowledge that through this way there is a better chance of success than through "blind" method.

Echoreflection is obtainable carving a few centimetre of the cathetere's tip or inserting a metallic component lengthways the said tip and at the same time a mandrel which allows both utilising soft plastic and having a memory effect.

The biological material preferably comprises seminal fluid or fluids in which are suspended pre-embryos or embryos.

A further object of the invention is a closed system provided with a thermostat and comprising the device, according to the present invention, in a computer based form for preparing motile spermatozoa and which is able to do the following steps:
a. aspiration of a fixed culture medium volume and successively seminal fluid in such a way as to form two layers;
b. inclination of the device at 45° and incubation at 37°C for 60 minutes;
c. ejection of seminal fluid.

The culture medium left into the device holds motile sperm which is maintained at 37°C until the use.

The related system can be advantageously used in the field of assisted reproduction techniques with particular reference to *in vivo* insemination techniques, i.e. intrauterine, intratubal and intraperitoneal.

### Description of the drawings

The present invention will be now described, for illustrative but not limitative purposes, according to its preferred embodiments, with particular reference to the figures of the enclosed drawings, wherein:
Figure 1 is a longitudinal section view of a first embodiment of the device according to the invention;
Figure 2a is a partially longitudinal section view of the piston of the device according to figure 1;
Figure 2b is a cross-section view taken along line B-B of figure 2a;
Figure 2c is a cross-section view taken along line C-C of figure 2a;
Figure 3a is a rear view of the ring nut of the device of figure 1;
Figure 3b is a longitudinal section view taken along line B-B of figure 3a;
Figure 3c is a longitudinal section view taken along line A-A of figure 3a;
Figure 4a is a rear view of the plug of the device of figure 1;
Figure 4b is a longitudinal section view taken along line A-A of figure 4a;
Figure 4c is a longitudinal section view taken along line B-B of figure 4a;
Figure 5 is a partially longitudinal section view of a second embodiment of the device according to the invention;
Figure 6 is a longitudinal section view taken along line A-A of figure 5; and
Figure 7 is a longitudinal section view taken along line B-B of figure 5.

### Detailed description of the invention

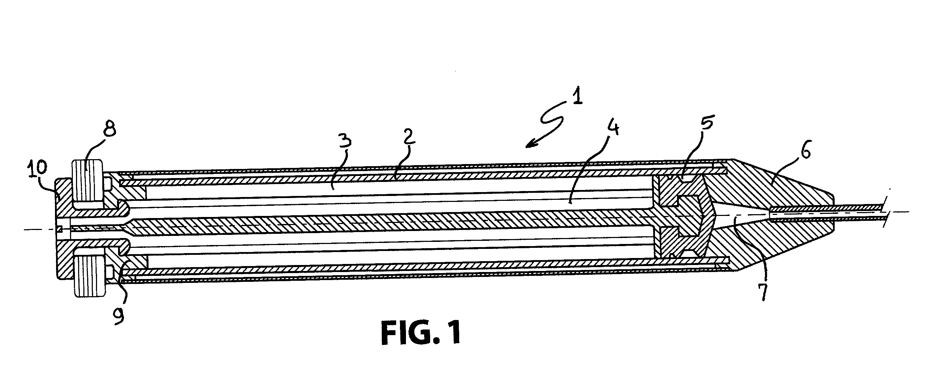

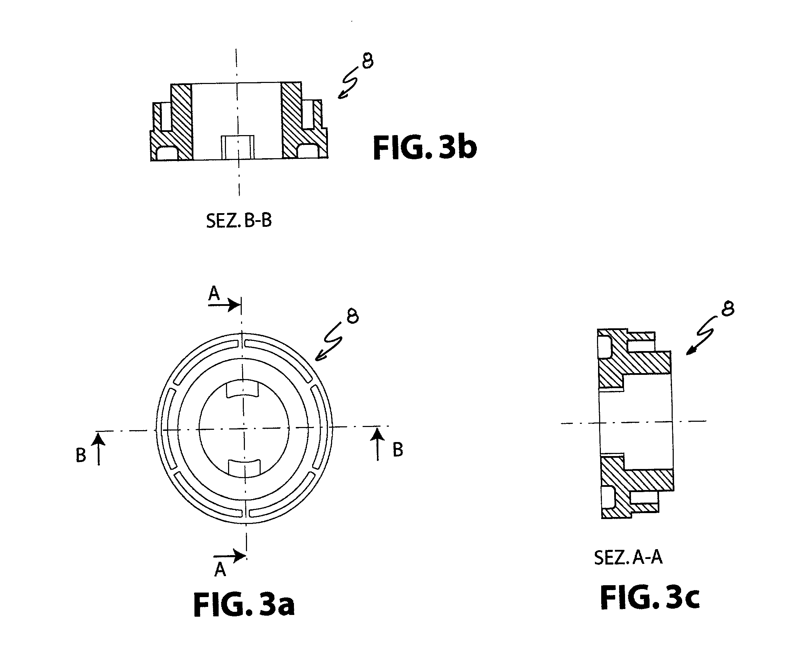

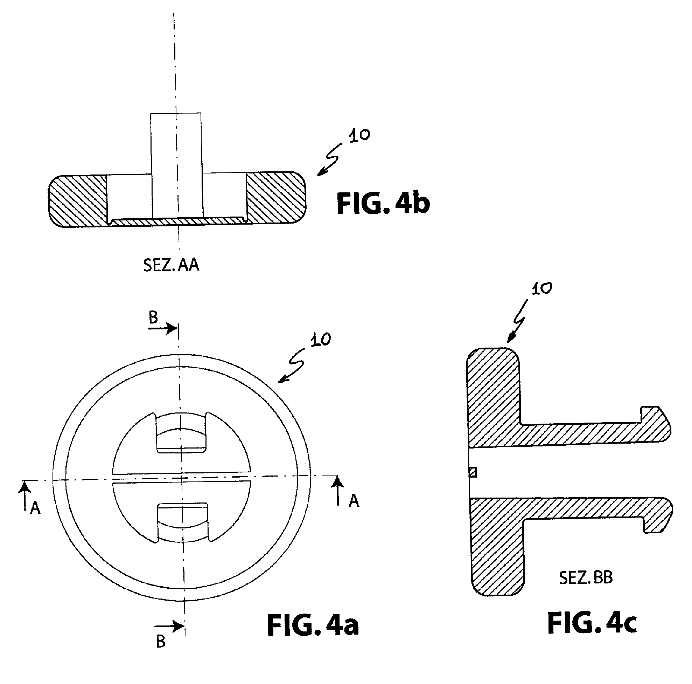

Observing first figures 1 - 4 of the enclosed drawings, it is shown a first embodiment of the device according to the invention, generically indicated by reference number 1, in this case a device directly comprising the disposable syringe.

Said device 1, that as already said, has the classic configuration of a syringe, has a cylindrical central body 2, a chamber 3, within said body 2, and within which a piston 4 slides, said piston having a front sealing element 5.

Said body 1 is tapered in the front portion indicated by reference number 6, and provides a hole 7, in correspondence of which a catheter 6 is coupled.

At the rear, said device 1 provides a structure allowing to obtain the results that are the basis of the solution according to the present invention.

Particularly, outside the piston 4 a rotating ring nut 8 is coupled by co-operating threads.

Between the ring nut 8 and the piston 4 a positioning element 9 is placed, provided with two fingers, acting along with the elastic ends of an end element 10.

As already said, the ring nut 8 is centrally holed, having a thread coupling with a corresponding thread provided outside said piston 4.

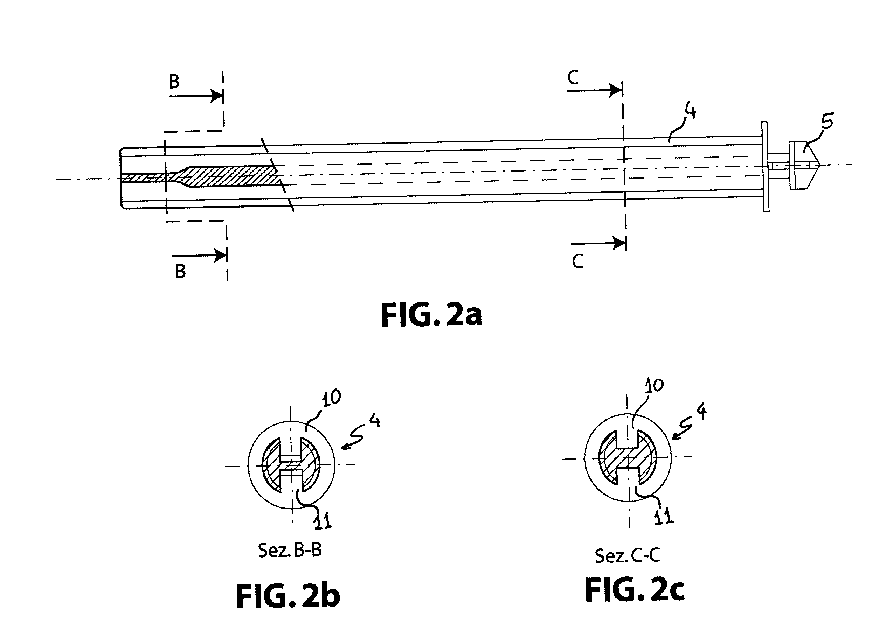

As it can be better observed from figures 2a - 2c, said piston provides two grooves 11 and 12, opposed each other, coupling with the two fingers of the positioning element 9, with the elastic ends of the end element 10, to prevent the rotation of the piston during its advancement.

Said ring nut 8 is rotatably coupled on the end element 10, in such a way to make the piston 4 longitudinally sliding within the chamber 3 and through the positioning element 9 and the end element 10.

Acting on the ring nut 8 it is possible to determine the optimum advancement of the piston 4, being in this way it possible to properly make the intrauterine transfer of biological material.

In a practical realization form of the device illustrated in the figures 1 to 4, the device 1 of 116,898 mm² in volume has been carried out, with a chamber 3 total volume of 9,352 ml in volume, piston stroke 4 of 80 mm in length, one turn of the nut 8 which coincide with a piston feed of 4,28 mm in length, injected volume for mm is about 0,117 ml, while injected volume for each nut turn is about 0,5 ml.

The device 1 according to the present invention is integral with the aspiration/ejection system, sterile, easy to make, having atraumatic
and echoreflecting catheter. Furthermore, it consists of nontoxic material, with the opportunity to be diversified for the different uses.

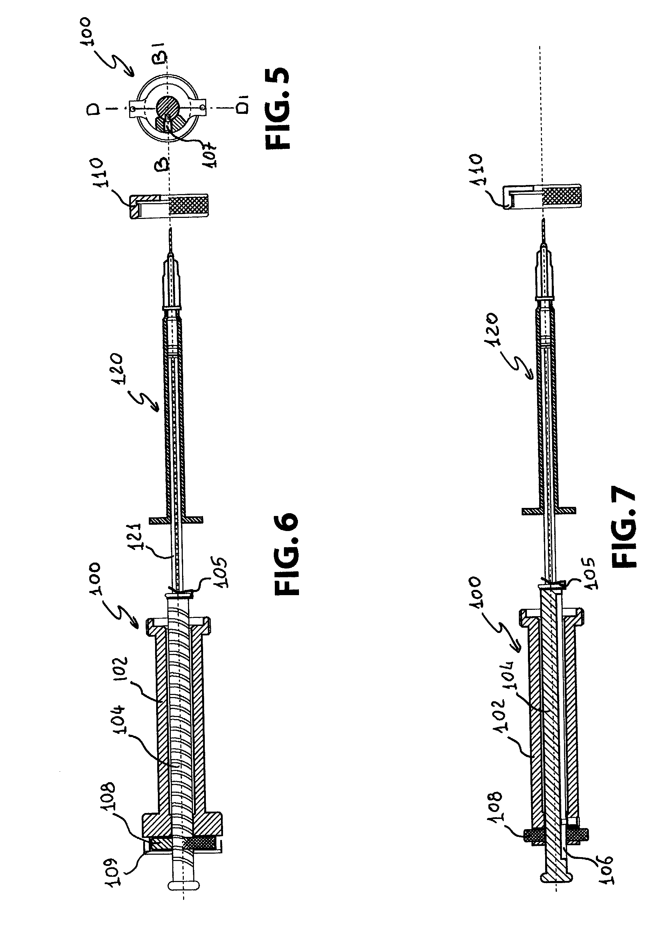

Now, examining the figures 5 to 7, it is shown a second embodiment of the device according to the present invention, generically indicated with reference number 100.

In this case, the device 100 according to the present invention is carried out in order to be fitted on the disposable syringes 120 on hand for the intrauterine transfer of biological material.

As one can see from the figures 5 to 7, the device 100 allows coupling between the syringe 120 and the system of aspiration/ejection of the material, by means of screw micrometric control.

Said device provides a central cylindrical body 102, or "dosage body", within which a piston, or "threaded trusting means" 104, having a plurality of principi, is provided. Number of thread principi and its pitch can be obviously modified in order to obtain the suction and outlet speed in function of the volume of the syringe 120 used.

On the end of the piston 104 toward the syringe 120 an elastic clip 105 is provided, for coupling the outer end of the piston 121 of the syringe 120.

Furthermore, said piston or "threaded trusting means" 104 has a groove or "channel guide" 106, within which a "guide screw" 107 is positioned, in such a way to allow the advancement without rotation about its longitudinal axis, transmitting the same motion to the piston 121 of the syringe 120 coupled with the same.

The other end of the piston 104 is coupled with a ring nut 108, or "dosage rotating ghera". Said ring nut 108 is innerly threaded, said thread coupling with a corresponding thread realised on the outer surface of the piston 104.

Positioning of the ring nut 108 is obtained by two spacing means 109.

As to the coupling between piston 104 and piston 121 of the syringe 120, beside the clip 105, a blocking ring nut 110 is provided, centrally holed for the insertion about the syringe 120, thus blocking the coupling between syringe 120 and device 100.

Rotating the ring nut 108, the axial motion of the piston 104 is obtained.

As it is evident, the solution of the second embodiment can be used employing standard syringes having different volumes, being only necessary to provide replacing parts suitable for the different volumes, namely the clip for coupling with the stem and the blocking ring nut 110.

Generally speaking, it stands to reason the importance of the disposable material in the medical practices, expecially in the outpatient ones. Indeed, in the case of recycled materials, there is also contamination risk of biological material to be transferred in addition to the already existing potential transmission risk. So, the use must be absolutely restricted once.

Sterility is an essential requirement for all clinical and laboratory materials being used in the PMA techniques.

The catheter must be sterilised in a separate package which must contain all the parts the catheter consists of. If the use of catheter provides different parts to be used subsequently, these latter should be sterilised and packaged separately and then joined in a single package, which is sterilised in its turn.

The easiness of use is considered as the minimum number of parts and the minimum acts to be done.

The catheter must be atraumatic both for embryos and spermatozoa it contains, and for uterine structures such as canalis cervicis, ostium internum and endometrium.

The atraumatism can be achieved through the fair choice of the plastic material and of the catheter's shape, and in particular of its tip. A round tip with side apertures is better for insemination, while it is not evaluated the same for embryos transfer yet.

Nevertheless, the embryos ejection system is also important since, with the existing available systems where the same embryos are loaded up a catheter with a diameter inferior to insulin syringe, it is evident that action exerted on the syringe piston, which often has a jerkily and not progressive feed, will shoot embryos into uterus cavity.

Non-toxicity must be evaluated in relation to chosen plastics and their reactions with the different culture medium used to transfer spermatozoa and embryos.

The possibility to display the catheter through ultrasounds allows to carry out uterine transfer under echography guide. There are many works that indicate greater results using this method in terms of pregnancy, in comparison to results obtained using "blind" method.

Echoreflection is obtainable carving a few centimetre of the cathetere's tip or inserting a metallic component lengthways the said tip and at the same time a mandrel which allows both utilising soft plastic and having a memory effect.

It is likely, that the choice to use till now an insulin syringe as an aspiration/ejection system, has been done in the view point to use a material which is already on the market, available and at low cost. Nowadays all the commercial catheters adopt this system.

Nevertheless the integral system with the catheter is a development as it allows to have a system without a break and to have a more "delicate" loading/espulsion mechanism.

The device can be diversified on the base of its use, that is to be provided with a catheter for insemination with side apertures, for embryos transfer with aperture on the tip, with or without a mandrel, or with a balloon for tubal perfusion.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Device for the intrauterine and/or intratubal and/or intraperitoneal transfer of biological material **characterised by** the fact that it comprises a syringe element, provided with a piston and means for drawing and introducing biological material, rotative means for shifting said piston of syringe element, and restraint means for shifting the piston, thus allowing a linear and non rotative shifting of the same piston.

2. Device according to claim 1, **characterised in that** it comprises the syringe body, within which a chamber is created, along said chamber a piston sliding, in front of said piston a catheter or similar element is provided, and provided, at the opposite end, with a threaded ring nut, rotatably mounted on said piston and acting with a corresponding thread provided outside said piston, and restraint means for the piston sliding, preventing the rotation under the ring nut action, thus allowing only its longitudinal sliding along the chamber of said sirynge.

3. Device according to claim 2, **characterised in that** at the rear of said chamber of the syringe body, a positioning element is provided, having step means, acting with elastic fingers realised in a end element, provided outside the ring nut with respect to said syringe body and passing within the same ring nut.

4. Device according to claim 1, **characterised in that** it provides a body, within which an actuating element or piston slides, that can be coupled in front of the rear end of a standard syringe piston, and couple at the opposite end with a dosage rotating ring nut, by interaction between corresponding threads, means being provided preventing the rotation of the actuating element with respect to the body due to the ring nut action, thus allowing only its longitudinal advancement.

5. Device according to claim 4, **characterised in that** coupling between the front end of the actuating element and the rear end of the syringe piston is obtained by an elastic clip.

6. Device according to claim 4 or 5, **characterised in that** it is provided a coupling ring nut between the front end of the actuating element and the rear end of the syringe piston, that is inserted outside the syringe and block said elastic clip.

7. Device according to claim 6, **characterised in that** said rotating dosage ring nut is maintained in position by plate means.

8. Device according to one of the claims 4 - 7, **characterised in that** said actuating element is provided with a longitudinal groove, interacting with pin means or like, sliding within the same, thus preventing the rotation of the actuating means due to the action of the ring nut, and allowing only its longitudinal sliding.

9. Device according to one of the preceding claims, **characterised in that** a different kind of coupling ring nut and elastic clip is provided in function of the kind of standard syringe.

10. Device according to one of the preceding claims, **characterised in that** the catheter is provided with round shaped tip having side apertures to reduce shocks risk both to uterine structures such as canalis cervicis, ostium internum and endometrium, and to biological material ejected from the syringe, since the peculiar conformation of the tip with side leakages reduces the pressures under which biological material is submitted, in comparison with a catheter having frontal aperture.

11. Device according to claim 10, **characterised in that** the catheter includes echoreflecting material which allows to display the catheter through ultrasounds and then to carry out uterine transfer under echography guide.

12. Device according to claim 10 or 11, **characterised in that** Echoreflection is obtained carving a few centimetre of the cathetere's tip or inserting a metallic component lengthways the said tip and at the same time a mandrel which allows both utilising soft plastic and having a memory effect.

13. Closed system provided with a thermostat and comprising the device, according to one of the preceding claims, automatic for preparing motile spermatozoa and which is able to do the following steps:
a. aspiration of a fixed culture medium volume and successively seminal fluid in such a way as to form two layers;
b. inclination of the device at 45° and incubation at 37°C for 60 minutes;
c. ejection of seminal fluid.
